# EUROPEAN PATENT APPLICATION

(11) **EP 1 317 924 A1**
(43) Date of publication of application: **11.06.2003**
(21) Application number: 02258123.5
(22) Date of filing: 26.11.2002
(51) Int. Cl.: A61K 31/505, A61P 21/00, A61P 29/02, A61P 43/00

(54) **Kit for reducing aching caused by pde-v inhibitors**

(30) Priority: 06.12.2001 GB 0129274
(71) Applicant: Pfizer Limited, Sandwich, Kent CT13 9NJ (GB); PFIZER INC., New York, N.Y. 10017 (US)
(72) Inventor: Abel, Samantha, Sandwich, Kent CT13 9NJ (GB); Ellis, Peter, Sandwich, Kent CT13 9NJ (GB)
(74) Representative: Wood, David John

(57) **Abstract**

The present invention relates to kits, and aspects thereof, for reducing or eliminating the aching associated with the administration of multiple doses of a PDE5 inhibitor, the kits comprising a plurality of pharmaceutical compositions for sequential administration over a period of time which compositions comprise increasing amounts of PDE5 inhibitor starting with an amount which gives a sub-optimal response and ending with an amount which gives an optimal response.

## Description

The present invention relates to kits, and aspects thereof, for the sequential administration of cyclic guanosine 3', 5'-monophosphate phosphodiesterase Type 5 inhibitors, by the use of which kits the aching associated with the multiple dosing of such inhibitors is eliminated or significantly reduced.

Compounds which inhibit cyclic guanosine 3', 5'-monophosphate phosphodiesterase Type 5, commonly referred to as cGMP PDE5 inhibitors or simply PDE5i's, such as those described in International Patent Application No. WO 94/28902, have been found to be potent and effective compounds for the treatment of male erectile dysfunction (MED), impotence and female sexual disorders. This finding led to the development of the compound sildenafil (5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one) (Viagra™) which has proved outstandingly successful as the first orally effective treatment for MED.

Following the success of sildenafil in treating MED, PDE5 inhibitors have been shown to have efficacy in the treatment of other medical conditions, such as neuropathy (EP 1129706), diabetic ulcers (WO 01/51042) and pulmonary hypertension (EP 1097711).

While MED has been found to be effectively treated by a single dose immediately prior to sexual activity, other diseases, such as those mentioned above, typically require a prolonged course of PDE5i treatment. This can involve dosing with a PDE5 inhibitor up to 3 (tid) or 4 times a day over a period of several days.

Examples of conditions for which multiple daily dosing of a PDE5 inhibitor is indicated include the aforementioned neuropathy, diabetic ulcers (particularly diabetic foot ulcers) and pulmonary hypertension plus conditions selected from the group comprising diabetes, hypertension, angina (including vasospastic angina), heart failure, intermittent claudication; post-angioplasty stenosis; cardioprotection (ischaemic preconditioning); atherosclerosis; acute coronary syndromes; chronic obstructive pulmonary disease (COPD); corpulmonale; Eisenmenger's syndrome; Raynaud's syndrome; systemic scleroderma; pre-eclampsia; intrauterine growth retardation (IUGR); infertility; preterm labour, recurrent abortion, dysmenorrhoea, stroke, Alzheimer's disease, cognitive impairment, Parkinson's and other degenerative disorders, glaucoma, macular degeneration, myasthenia gravis, benign prostatic hyperplasia (BPH), lower urinary tract syndrome (LUTS)/overactive bladder (OAB), chronic inflammatory hyperalgesia, neuropathic pain, back pain, chronic lymphatic leukaemia (CLL)/apoptosis, insulin resistance syndrome, acne and anal fissures

While the foregoing are all non-sexual conditions, it is possible to envisage a situation wherein multiple dosing of a PDE5 inhibitor might be useful in the long-term treatment of persistent sexual conditions, for example, the aforementioned MED and female sexual disorders. The latter include female sexual dysfunction (FSD), clitoral dysfunction, female sexual arousal disorder, female sexual pain disorder or female sexual orgasmic dysfunction (FSOD), sexual dysfunction due to spinal cord injury and selective serotonin reuptake inhibitor (SSRi)-induced sexual dysfunction.

A problem associated with the multiple daily dosing of PDE5 inhibitors is that subjects experience, as a side effect, aching in certain parts of the body. Subjects describe this aching as predominantly a dull aching without muscle tenderness. Aching is experienced predominantly in the "postural muscles", typically the calves, thighs, buttocks, lower back, shoulders and neck regions. These symptoms may be described as myalgia or back pain; for the purposes of this application, they will be referred to as "aching". Often the aching is painful and debilitating and in some individuals can result in their stopping a course of treatment with a PDE5 inhibitor.

Aching as a side effect appears to be a class effect associated with the multiple dosing of any PDE5 inhibitor. With sildenafil, aching generally commences on the second or third day of a multiple dosing regime. At higher doses (for example, 75mg tid), there is evidence that aching disappears by the sixth or seventh day. At higher doses (75mg tid), the incidence of aching was 100%, falling to 60% at a dose of 25mg tid. The effect has also been observed with the PDE5 inhibitors 3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]-pyrimidin-7-one (WO 99/54333, Example 4) and 3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (WO 98/49166, Example 4). The PDE5 inhibitor tadalafil (Cialis™) has also been reported to cause backache and myalgia (H Padma-Nathan *et al*, Int. J. Impotence Res., 2001, 12, 2-9 and the presentation by Eli Lilly/ICOS at the European Society for Sexual and Impotence Research meeting held in Rome between 30 September 2001 and 3 October 2001).

We have now unexpectedly found that the severity of the aching associated with multiple dosing of PDE5 inhibitors may be significantly reduced by administering the PDE5 inhibitor in a defined sequence over a period of time, specifically by starting with an amount of PDE5 inhibitor which causes a sub-optimal response and ending with an amount which causes an optimal response. The amount of PDE5 inhibitor used to give the sub-optimal response is typically from 1 to 50%, preferably from 1 to 25%, of the amount which gives the optimal response, optimal response being defined as the desired clinical response with a minimum of non-aching side effects.

This finding was particularly surprising as sequential administration has previously been applied to relieve acute side effects experienced after a first single dose. Sequential administration would not have been expected to limit side effects which were experienced after several days, since late onset side effects are due to overall total exposure to drug over time, rather than the typical first exposure side effects which are directly associated with the plasma concentration following a high first dose.

We have also surprisingly found that when the subject is multiply dosed in accordance with the invention, if the patient misses one or more doses (or even an entire daily dose) and resumes taking the PDE5 inhibitor at the same dose, aching does not re-occur.

Sequential administration in accordance with the invention of a PDE5 inhibitor selected from sildenafil, 3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (WO 99/54333, Example 4), 3-ethyl-5-[5-(4-ethylpiperazin-1-ylsulphonyl)-2-*n*-propoxyphenyl]-2-(pyridin-2-yl)methyl-2,6-dihydro-7H-pyrazolo[4,3-d]pyrimidin-7-one (WO 98/49166, Example 4) and 2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) has indicated that for the treatment of neuropathy, diabetic ulcers (particularly diabetic foot ulcers), pulmonary hypertension and diabetes the amount of PDE5 inhibitor giving a sub-optimal response is typically in the range of from 1 to 25mg and the amount which causes an optimal response is in the range of from 25 to 100mg.

According to a first aspect of the invention, there is provided a kit for reducing the aching associated with the administration of multiple doses of a PDE5 inhibitor, said kit comprising a plurality of pharmaceutical compositions for sequential administration over a period of time which compositions comprise increasing amounts of PDE5 inhibitor starting with an amount which gives a sub-optimal response and ending with an amount which gives an optimal response.

According to three preferred embodiments of the first aspect of invention, said kit ideally comprises
(i) from 2 to 6 PDE5i compositions for sequential administration over 2 days wherein the amount in those compositions which give a sub-optimal response comprises from 1 to 50%, preferably from 1 to 25%, of the amount which gives an optimal response;
(ii) from 14 to 42 PDE5i compositions for sequential administration over 14 days wherein the amount in those compositions which give a sub-optimal response comprises from 1 to 50%, preferably from 1 to 25%, of the amount which gives an optimal response; and
(iii) compositions comprising (a) from 1 to 15mg sildenafil for administration from 1 to 3 times a day for 3 to 14 days and (b) compositions comprising from 25 to 100mg of sildenafil for administration for the remainder of the prescribed treatment period.

By "prescribed treatment period" is meant the period that the physician deems it necessary to administer the drug at therapeutically effective doses.

According to another aspect of the invention, there is provided the use of a PDE5 inhibitor in the manufacture of a pharmaceutical composition for the treatment of any condition for which a PDE5 inhibitor is indicated, which composition is one of the plurality of pharmaceutical compositions used in the kit of the invention.

Preferred embodiments of this use include the manufacture of pharmaceutical compositions in accordance with those found in the preferred embodiments of the kit *(supra)* for the treatment of one or more of the aforementioned conditions.

According to a further aspect of the invention, there is provided a method for the treatment of any condition for which multiple dosing of a PDE5 inhibitor is indicated, which method comprises the sequential administration of a plurality of pharmaceutical compositions over a period of time which compositions are in accordance with those used in the kit of the invention.

Preferred embodiments of this method of treatment include those methods which employ a plurality of compositions in accordance with those found in the preferred embodiments of the kit *(supra)* in the treatment of one or more of the aforementioned conditions.

In all of the aforementioned aspects of the invention, the PDE5 inhibitor which gives the sub-optimal response may be different from the PDE5 inhibitor which gives the optimal response.

PDE5 inhibitors suitable for use in all aspects of the invention include
pyrazolo[4,3-d]pyrimidin-7-ones as disclosed in EP 0463756;
pyrazolo[4,3-d]pyrimidin-7-ones as disclosed in EP 0526004;
pyrazolo[4,3-d]pyrimidin-7-ones as disclosed in WO 93/06104;
isomeric pyrazolo[3,4-d]pyrimidin-4-ones as disclosed in WO 93/07149;
quinazolin-4-ones as disclosed in WO 93/12095;
pyrido[3,2-d]pyrimidin-4-ones as disclosed in WO 94/05661;
purin-6-ones as disclosed in WO 94/00453;
pyrazolo[4,3-d]pyrimidin-7-ones as disclosed in WO 98/49166;
pyrazolo[4,3-d]pyrimidin-7-ones as disclosed in WO 99/54333;
pyrazolo[4,3-d]pyrimidin-4-ones as disclosed in EP 0995751;
pyrazolo[4,3-d]pyrimidin-7-ones as disclosed in WO 00/24745;
pyrazolo[4,3-d]pyrimidin-4-ones as disclosed in EP 0995750;
compounds disclosed in WO 95/19978;
compounds disclosed in WO 99/24433;
compounds disclosed in WO 93/07124;
pyrazolo[4,3-d]pyrimidin-7-ones as disclosed in WO 01/27112;
pyrazolo[4,3-d]pyrimidin-7-ones as disclosed in WO 01/27113;
compounds disclosed in EP 1092718; and
compounds disclosed in EP 1092719.

Specific PDE5 inhibitors suitable for use in all aspects of the invention include
4-bromo-5-(pyridylmethylamino)-6-[3-(4-chlorophenyl)-propoxy]-3(2H)pyridazinone;
1-[4-[(1,3-benzodioxol-5-ylmethyl)amino]-6-chloro-2-quinozolinyl]-4-piperidine-carboxylic acid, monosodium salt;
(+)-*cis*-5,6a,7,9,9,9a-hexahydro-2-[4-(trifluoromethyl)-phenylmethyl-5-methyl-cyclopent-4,5]imidazo[2,1-b]purin-4(3H)one;
furazlocillin;
cis-2-hexyl-5-methyl-3,4,5,6a,7,8,9,9a-octahydrocyclopent[4,5]-imidazo[2,1-b]purin-4-one;
3-acetyl-1-(2-chlorobenzyl)-2-propylindole-6-carboxylate;
3-acetyl-1-(2-chlorobenzyl)-2-propylindole-6-carboxylate;
4-bromo-5-(3-pyridylmethylamino)-6-(3-(4-chlorophenyl)propoxy)-3-(2H)pyridazinone;
l-methyl-5(5-morpholinoacetyl-2-n-propoxyphenyl)-3-n-propyl-1,6-dihydro-7H-pyrazolo(4,3-d)pyrimidin-7-one;
1-[4-[(1,3-benzodioxol-5-ylmethyl)amino]-6-chloro-2-quinazolinyl]-4-piperidinecarboxylic acid, monosodium salt;
compounds of Pharmaprojects No. 4516 (Glaxo Wellcome);
compounds of Pharmaprojects No. 5051 (Bayer);
compounds of Pharmaprojects No. 5064 (Kyowa Hakko; WO 96/26940);
compounds of Pharmaprojects No. 5069 (Schering Plough);
GF-196960 (Glaxo Wellcome);
E-8010 and E-4010 (Eisai);
Bay-38-3045 & 38-9456 (Bayer); and
Sch-51866 (Schering Plough).

Particularly preferred PDE5 inhibitors suitable for use in all aspects of the invention include
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil), also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (EP 0463756);
5-(2-ethoxy-5-morpholinoacetylphenyl)-1-methyl-3-n-propyl-1,6-dihydro-7H-pyrazolo[4,3-d]pyrimidin-7-one (EP 0526004);
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (WO 95/19978, Examples 1, 3, 7, 8, 78 and 95) (tadalafil);
2-[2-ethoxy-5-(4-ethyl-piperazin-1 -yl-1-sulphonyl)-phenyl]-5-methyl-7-propyl-3H-imidazo[5,1-f][1,2,4]triazin-4-one, also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine (WO99/24433, Examples 20, 19, 336 and 337) (vardenafil);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (WO 01/27113, Example 8);
5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one (WO 01/27112, Example 132);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (WO 99/54333, Example 4);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (WO 98/49166, Example 4);
(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1 R)-2-methoxy-1-methylethyl]oxy)pyridin-3-yl}-2-methyl-2,6-dihydro-7H-pyrazolo[4,3-d] pyrimidin-7-one (WO 99/54333);
5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (WO 01/27113, Example 15);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (WO 01/27113, Example 66);
5-(5-Acetyl-2-propoxy-3-pyridinyl)-3-ethyl-2-(1-isopropyl-3-azetidinyl)-2,6-dihydro-7H-pyrazolo[4,3-*d*]pyrimidin-7-one (WO 01/27112, Example 124); the compound of Example 11 of International Patent Application No. WO 93/07124 (Eisai); and
compounds 3 and 14 of Rotella D P, *J Med Chem,* 43, 1257 (2000).

The contents of the published patent applications and journal articles, particularly the general formulae of the therapeutically active compounds of the claims and compounds exemplified therein, are incorporated herein by reference.

Of the aforementioned compounds,
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil);
5-(2-ethoxy-5-morpholinoacetylphenyl)-1-methyl-3-n-propyl-1,6-dihydro-7H-pyrazolo[4,3-d]pyrimidin-7-one;
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (tadalafil);
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one;
5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one;
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one;
3-ethyl-5-[5-(4-ethylpiperazin-1-ylsulphonyl)-2-*n*-propoxyphenyl]-2-(pyridin-2-yl)methyl-2,6-dihydro-7H-pyrazolo[4,3-d]pyrimidin-7-one);
and pharmaceutically acceptable salts thereof are particularly preferred.

And of these compounds,
5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one (sildenafil);
5-(2-ethoxy-5-morpholinoacetylphenyl)-1-methyl-3-*n*-propyl-1,6-dihydro-7H-pyrazolo[4,3-d]pyrimidin-7-one;
and pharmaceutically acceptable salts thereof are especially preferred, particularly the citrate of the former.

The following Examples derived from studies involving the sequential administration of the PDE5 inhibitors sildenafil and 5-(2-ethoxy-5-morpholinoacetylphenyl)-1-methyl-3-*n*-propyl-1,6-dihydro-7H-pyrazolo[4,3-d]pyrimidin-7-one (Compound A) serve to illustrate the invention.

### EXAMPLES

All adverse events observed or recorded during the studies were recorded by the investigator, including information about concomitant illnesses and therapeutic interventions. For all adverse events, the investigator pursued and obtained information adequate to determine the outcome of the adverse event. Physical examinations at screening and medical histories of all subjects were normal with no indication of any joint, muscle, or tendon weakness.

The Examples typically commence at a low dose of PDE5 inhibitor, for example, 2.5mg three times a day (tid) for Compound A (Examples 4, 6 and 7) with periodic dose escalation aimed at achieving a final regimen of 20mg tid. By means of such dose escalation, it was possible to significantly reduce the aching previously associated with the multiple dosing of PDE5 inhibitors.

### STUDY 1 - SILDENAFIL

### Example 1

### (Placebo and comparative - negative and positive controls)

In a double blind, placebo-controlled, multiple dose study, from 8 to 10 subjects were given 5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one (sildenafil) three times a day at one of three different doses (25mg tid, 50mg tid, or 75mg tid). Dosage took the form of oral capsules.

The most common adverse events were back pain, headache and myalgia.

The results for myalgia and back pain ("aching") were as follows:

**TABLE 1**

| Dosage | Placebo | 25mg tid | 50mg tid | 75mg tid |
|---|---|---|---|---|
| Myalgia | 0/8 | 2/10 | 5/9 | 3/8 |
| Back pain | 0/8 | 2/10 | 2/9 | 5/8 |

At the lower doses (25mg tid and 50mg tid), headache and myalgia typically occurred after a few days of treatment, but did not persist with further treatment.

At the higher dose (75mg tid), there were three reports of severe treatment-related adverse events which led to discontinuance of the treatment for the subjects in question. These subjects experienced respectively (1) severe myalgia (lower back pain) on Day 27 after 5 days of tid dosing, treatment discontinued on Day 30, (2) severe lower back pain on Day 27 after 4 days of tid dosing, treatment discontinued on Day 31 and (3) severe pain (throbbing pain behind thighs) on Day 23 after 2 days of tid dosing, treatment discontinued on Day 24.

### STUDY 2 - COMPOUND A

### Example 2

### (Placebo - negative control)

Nine subjects were given placebo three times a day (tid) for 21 days.

None of the subjects reported any myalgia or back pain ("aching"):

**TABLE 2**

| | Mild/moderate | Severe |
|---|---|---|
| Myalgia/back pain | 0/9 | 0/9 |

### Example 3

### (Comparative - positive control)

Six subjects were given 20mg of Compound A three times a day (tid) for 7 days. Dosage took the form of immediate release tablets.

The results for myalgia and back pain ("aching") were as follows:

**TABLE 3**

| | Mild/moderate | Severe |
|---|---|---|
| Myalgia/back pain | 0/6 | 6/6 |

Due to the severity of the aching, four subjects had to be withdrawn after only 4 days of treatment.

### Example 4

Nine subjects were given 2.5mg of Compound A three times a day (tid) for 7 days, then 7.5mg tid for 7 days, then 20mg tid for a further 7 days. Dosage took the form of immediate release tablets.

The results for myalgia and back pain ("aching") were as follows:

**TABLE 4**

| | Mild/moderate | Severe |
|---|---|---|
| Myalgia/back pain | 1/9 | 1/9 |

### CONCLUSION

It can be seen from Table 4 that those subjects who followed the escalating dosing regime of Example 4 experienced a significant reduction in aching and had no withdrawals compared with those subjects who followed the non-escalating dosing regime of (comparative) Example 3.

### STUDY 3 - COMPOUND A

### Example 5

### (Placebo - negative control)

Nine subjects were given placebo three times a day (tid) for 21 days.

The results for myalgia and back pain ("aching") were as follows:

**TABLE 5**

| | Mild | Moderate | Severe |
|---|---|---|---|
| Myalgia | 4/9 | 0/9 | 0/9 |
| Back pain | 0/9 | 0/9 | 0/9 |

### Example 6

Nine subjects were given 2.5mg of Compound A three times a day (tid) for 7 days followed by 20mg tid for a further 7 days. The subjects were given a 24-hour drug holiday, *i.e.* a day without taking the inhibitor, then 20mg tid dosing was continued for another 6 days. Dosage took the form of immediate release tablets.

The results for myalgia and back pain ("aching") were as follows:

**TABLE 6**

| | Mild | Moderate | Severe |
|---|---|---|---|
| Myalgia | 2/9 | 3/9 | 1/9 |
| Back pain | 2/9 | 0/9 | 0/9 |

There was no apparent increase in the incidence of aching after the 24-hour drug-free period.

### Example 7

Nine subjects were given 2.5mg of Compound A three times a day (tid) for 4 days, then 7.5mg tid for 3 days, then 20mg tid for a further 7 days. The subjects were given a 24-hour drug holiday, *i.e.* a day without taking the inhibitor, then 20mg tid dosing was continued for another 6 days. Dosage took the form of immediate release tablets.

The results for myalgia and back pain (" aching") were as follows:

**TABLE 7**

| | Mild | Moderate | Severe |
|---|---|---|---|
| Myalgia | 4/9 | 1/9 | 0/9 |
| Back pain | 0/9 | 0/9 | 0/9 |

There was no apparent increase in the incidence of aching after the 24-hour drug-free period.

### CONCLUSION

It can be seen from Tables 6 and 7 that those subjects who followed the escalating dosing regimes of Examples 6 and 7 experienced a significant reduction in aching and had no withdrawals compared with those subjects who followed the non-escalating dosing regime of (comparative) Example 3. Triple dosing (Example 7) appeared to be particularly effective in reducing aching.

It was also observed that the 24-hour drug-free period of Examples 6 and 7 did not lead to an increased incidence of aching when 20mg tid dosing was resumed. Thus strict compliance with the escalating dosing regime does not appear to be necessary in order to achieve the desired effect.

## Claims

1. A kit for reducing the aching associated with the administration of multiple doses of a PDE5 inhibitor, said kit comprising a plurality of pharmaceutical compositions for sequential administration over a period of time which compositions comprise increasing amounts of PDE5 inhibitor starting with an amount which gives a sub-optimal response and ending with an amount which gives an optimal response.

2. A kit according to Claim 1, wherein said kit comprises from 2 to 6 pharmaceutical compositions for sequential administration over 2 days which compositions comprise increasing amounts of PDE5 inhibitor starting with from 1 to 50% of the amount which gives an optimal response and ending with an amount which gives an optimal response.

3. A kit according to Claim 1, wherein said kit comprises from 14 to 42 pharmaceutical compositions for sequential administration over 14 days which compositions comprise increasing amounts of PDE5 inhibitor starting with from 1 to 50% of the amount which gives an optimal response and ending with an amount which gives an optimal response.

4. A kit according to Claim 2 or 3, wherein said compositions comprise increasing amounts of PDE5 inhibitor starting with from 1 to 25% of the amount which gives an optimal response and ending with an amount which gives an optimal response.

5. A kit according to any of Claims 1 to 4, wherein the PDE5 inhibitor which gives the sub-optimal response is different from the PDE5 inhibitor which gives the optimal response.

6. A kit according to any of Claims 1 to 5 wherein the PDE5 inhibitor(s) is one or more selected from the group comprising
5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one (sildenafil);
5-(2-ethoxy-5-morpholinoacetylphenyl)-1-methyl-3-n-propyl-1,6-dihydro-7H-pyrazolo[4,3-d]pyrimidin-7-one;
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (tadalafil);
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one;
5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one;
3-ethyl-5-[5-(4-ethylpiperazin-1-ylsulphonyl)-2-*n*-propoxyphenyl]-2-(pyridin-2-yl)methyl-2,6-dihydro-7H-pyrazolo[4,3-d]pyrimidin-7-one);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one;
and pharmaceutically acceptable salts thereof.

7. A kit according to Claim 6 wherein the PDE5 inhibitor is
5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one (sildenafil);
5-(2-ethoxy-5-morpholinoacetylphenyl)-1-methyl-3-n-propyl-1,6-dihydro-7H-pyrazolo[4,3-d]pyrimidin-7-one;
or a pharmaceutically acceptable salt of either thereof.

8. A kit for reducing the aching associated with the administration of multiple doses of sildenafil, said kit comprising a plurality of pharmaceutical compositions for sequential administration over 7 or more days which compositions comprise (a) from 1 to 15mg of sildenafil to be administered from 1 to 3 times a day for 3 to 14 days and (b) from 25 to 100mg of sildenafil to be administered for the remainder of the prescribed treatment period.

9. The use of a PDE5 inhibitor in the manufacture of a pharmaceutical composition for the treatment of any condition for which multiple dosing of said inhibitor is indicated, wherein said composition is one of a plurality of pharmaceutical compositions for sequential administration over a period of time which compositions comprise increasing amounts of PDE5 inhibitor starting from an amount which gives a sub-optimal response and ending with an amount which gives an optimal response.

10. Use according to Claim 9, wherein said composition comprises one of from 2 to 6 pharmaceutical compositions for sequential administration over a period of 2 days which compositions comprise increasing amounts of PDE5 inhibitor starting with an amount comprising from 1 to 50% of the amount which gives an optimal response and ending with an amount which gives an optimal response.

11. Use according to Claim 9, wherein said composition comprises one of from 14 to 42 pharmaceutical compositions for sequential administration over a period of 14 days which compositions comprise increasing amounts of PDE5 inhibitor starting with an amount comprising from 1 to 50% of the amount which gives an optimal response and ending with an amount which gives an optimal response.

12. Use according to Claim 10 or 11, wherein said compositions comprise increasing amounts of PDE5 inhibitor starting with an amount comprising from 1 to 25% of the amount which gives an optimal response and ending with an amount which gives an optimal response.

13. Use according to any of claims 9 to 12, wherein the PDE5 inhibitor which gives the sub-optimal response is different from the PDE5 inhibitor which gives the optimal response.

14. Use according to any of Claims 9 to 13, wherein the PDE5 inhibitor(s) is one or more selected from the group comprising
5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one (sildenafil);
5-(2-ethoxy-5-morpholinoacetylphenyl)-1-methyl-3-*n*-propyl-1,6-dihydro-7H-pyrazolo[4,3-d]pyrimidin-7-one;
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (tadalafil);
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one;
5-(5-acetyl-2-butoxy-3-pyridinyl)-3-ethyl-2-(1-ethyl-3-azetidinyl)-2,6-dihydro-7H-pyrazolo[4,3-*d*]pyrimidin-7-one;
3-ethyl-5-[5-(4-ethylpiperazin-1-ylsulphonyl)-2-*n*-propoxyphenyl]-2-(pyridin-2-yl)methyl-2,6-dihydro-7H-pyrazolo[4,3-d]pyrimidin-7-one);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one;
and pharmaceutically acceptable salts thereof.

15. Use according to Claim 14 wherein the PDE5 inhibitor is
5-[2-ethoxy-5-(4-methyl-1-piperazinylsulphonyl)phenyl]-1-methyl-3-*n*-propyl-1,6-dihydro-7H-pyrazolo[4,3-d]pyrimidin-7-one (sildenafil);
5-(2-ethoxy-5-morpholinoacetylphenyl)-1-methyl-3-n-propyl-1,6-dihydro-7H-pyrazolo[4,3-d]pyrimidin-7-one;
or a pharmaceutically acceptable salt of either thereof.

16. The use of sildenafil in the manufacture of a pharmaceutical composition for the treatment of any condition for which multiple dosing of said compound is indicated, wherein said composition is one of a plurality of pharmaceutical compositions for sequential administration over a period of 7 or more days and comprises either (a) from 1 to 15mg of sildenafil to be administered from 1 to 3 times a day for a period of from 3 to 14 days or (b) from 25 to 100mg of sildenafil to be administered for the remainder of the prescribed treatment period.

17. Use according to any of Claims 9 to 16, wherein said condition is selected from the group comprising neuropathy, diabetic ulcers, (particularly diabetic foot ulcers), pulmonary hypertension, diabetes, hypertension, angina (including vasospastic angina), heart failure, intermittent claudication, post-angioplasty stenosis, cardioprotection (ischaemic preconditioning), atherosclerosis, acute coronary syndromes, chronic obstructive pulmonary disease; corpulmonale; Eisenmenger's syndrome; Raynaud's syndrome, systemic scleroderma, pre-eclampsia, intrauterine growth retardation, infertility, preterm labour, recurrent abortion, dysmenorrhoea, stroke, Alzheimer's disease, cognitive impairment, Parkinson's and other degenerative disorders, glaucoma, macular degeneration, myasthenia gravis, benign prostatic hyperplasia, lower urinary tract syndrome/overactive bladder, chronic inflammatory hyperalgesia, neuropathic pain, back pain, chronic lymphatic leukaemia/apoptosis, insulin resistance syndrome, acne and anal fissures.

18. A method for the treatment of any condition for which multiple dosing of a PDE5 inhibitor is indicated, which method comprises the sequential administration of a plurality of pharmaceutical compositions over a period of time which compositions comprise increasing amounts of PDE5 inhibitor starting with an amount which gives a sub-optimal response and ending with an amount which gives an optimal response.

19. A method according to Claim 18, which method comprises the sequential administration of from 2 to 6 pharmaceutical compositions over 2 days which compositions comprise increasing amounts of PDE5 inhibitor starting with an amount comprising from 1 to 50% of the amount which gives an optimal response and ending with an amount which gives an optimal response.

20. A method according to Claim 18, which method comprises the sequential administration of from 14 to 42 pharmaceutical compositions over 14 days which compositions comprise increasing amounts of PDE5 inhibitor starting with an amount comprising from 1 to 50% of the amount which gives an optimal response and ending with an amount which gives an optimal response.

21. A method according to Claim 19 or 20, wherein said compositions comprise increasing amounts of PDE5 inhibitor starting with an amount comprising from 1 to 25% of the amount which gives an optimal response and ending with an amount which gives an optimal response.

22. A method according to any of Claims 18 to 21, wherein the PDE5 inhibitor which give the sub-optimal response is different from the PDE5 inhibitor which gives the optimal response.

23. A method according to any of Claims 18 to 22, wherein the PDE5 inhibitor(s) is one or more selected from the group comprising
5-[2-ethoxy-5-(4-methyl-1-piperazinylsulphonyl)phenyl]-1-methyl-3*-n*-propyl-1,6-dihydro-7H-pyrazolo[4,3-d]pyrimidin-7-one (sildenafil);
5-(2-ethoxy-5-morpholinoacetylphenyl)-1-methyl-3-*n*-propyl-1,6-dihydro-7H-pyrazolo[4,3-d]pyrimidin-7-one;
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (tadalafil);
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one;
5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one;
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one;
and pharmaceutically acceptable salts thereof.

24. A method according to Claim 23 wherein the PDE5 inhibitor is
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil);
5-(2-ethoxy-5-morpholinoacetylphenyl)-1-methyl-3-n-propyl-1,6-dihydro-7H-pyrazolo[4,3-d]pyrimidin-7-one;
or a pharmaceutically acceptable salt of either thereof.

25. A method for the treatment of any condition for which multiple dosing of sildenafil is indicated, which method comprises the administration over a period of seven or more days of (a) from 1 to 15mg of sildenafil from 1 to 3 times a day for 3 to 14 days and (b) the administration of from 25 to 100mg of sildenafil for the remainder of the prescribed treatment period.

26. A method according to any of claims 18 to 25, wherein said condition is selected from the group comprising neuropathy, diabetic ulcers, (particularly diabetic foot ulcers), pulmonary hypertension, diabetes, hypertension, angina (including vasospastic angina), heart failure, intermittent claudication, post-angioplasty stenosis, cardioprotection (ischaemic preconditioning), atherosclerosis, acute coronary syndromes, chronic obstructive pulmonary disease; corpulmonale; Eisenmenger's syndrome; Raynaud's syndrome, systemic scleroderma, pre-eclampsia, intrauterine growth retardation, infertility, preterm labour, recurrent abortion, dysmenorrhoea, stroke, Alzheimer's disease, cognitive impairment, Parkinson's and other degenerative disorders, glaucoma, macular degeneration, myasthenia gravis, benign prostatic hyperplasia, lower urinary tract syndrome/overactive bladder, chronic inflammatory hyperalgesia, neuropathic pain, back pain, chronic lymphatic leukaemia/apoptosis, insulin resistance syndrome, acne and anal fissures.
